# EUROPÄISCHE PATENTSCHRIFT

(11) **EP 1 512 368 B1**
(45) Veröffentlichungstag und Bekanntmachung des Hinweises auf die Patenterteilung: **27.06.2012**
(21) Anmeldenummer: 04015868.5
(22) Anmeldetag: 06.07.2004
(51) Int. Cl.: A61B 3/107, A61B 3/10

(54) **Ophthalmologisches Analysesystem**
Ophthalmological analysis system
Système d'analyse ophthalmologique

(30) Priorität: 02.09.2003 DE 20313745 U
(43) Veröffentlichungstag der Anmeldung: 09.03.2005
(73) Patentinhaber: Oculus Optikgeräte GmbH, 35582 Wetzlar-Dutenhofen (DE)
(72) Erfinder: Köst, Gert, Dipl.-Biologe, D-30171 Hannover (DE)
(74) Vertreter: von den Steinen, Axel

(56) Entgegenhaltungen:
- EP-A- 1 138 254
- EP-A- 1 138 257
- WO-A-03/032823
- US-A1- 2002 154 269
- HACHICHA A ET AL: "THE USE OF GRAY-LEVEL INFORMATION AND FITTING TECHNIQUES FOR PRECISE MEASUREMENT OF CORNEAL CURVATURE AND THICKNESS", COMPUTER VISION GRAPHICS AND IMAGE PROCESSING, ACADEMIC PRESS, DULUTH, MA, US, vol. 47, no. 2, 1 August 1989 (1989-08-01) , pages 131-164, XP000103822,
- KAMPFER T ET AL: "Improved biometry of the anterior eye segment", OPHTHALMIC RESEARCH, KARGER, CH, vol. 21, no. 3, 1 January 1989 (1989-01-01), pages 239-248, XP008142534, ISSN: 0030-3747, DOI: 10.1159/000266815
- OLSEN T ET AL: "On the optical measurement of a corneal thickness. I. Optical principle and sources of error", ACTA OPHTHALMOLOGICA SCANDINAVICA FOUNDATION, ACTA OPHTHALMOLOGICA SCANDINAVICA FOUNDATION, DENMARK, vol. 58, no. 5, 1 October 1980 (1980-10-01), pages 760-766, XP008142688, ISSN: 0001-639x, DOI: 10.1111/J.1755-3768.1980.TB06689.X [retrieved on 2009-05-27]
- RICHARDS D W ET AL: "A method for improved biometry of the anterior chamber with a Scheimpflug technique.", INVESTIGATIVE OPHTHALMOLOGY & VISUAL SCIENCE DEC 1988 LNKD- PUBMED:3192372, vol. 29, no. 12, December 1988 (1988-12), pages 1826-1835, ISSN: 0146-0404

## Beschreibung

Die Erfindung betrifft ein ophthalmologisches Analysesystem zur Messung der Dicke der Hornhaut an einem menschlichen Auge gemäß dem Oberbegriff des Anspruchs 1.

Derartige Analysesysteme sind in der Augenheilkunde von größter Bedeutung. Durch Einsatz entsprechend geeigneter Bildverarbeitungsverfahren können außerordentlich effektiv die signifikanten Eigenschaften des Hornhautgewebes festgestellt werden.

Untersuchungen haben ergeben, dass die Messung der Dicke der Hornhaut vom Krümmungsradius der Hornhaut im Messbereich abhängt. Dies bedeutet mit anderen Worten, dass die bislang bekannte Messung der Dicke des Hornhautgewebes einen Messfehler aufweist, je weiter die tatsächliche Krümmung der Hornhaut im Messbereich von dem in Analysesystem vorgegebenen Referenzwert abweicht.

Aus der EP 1 138 257 A1 ist ein ophthalmologisches Analysesystem bekannt, welches aus einem Pachymeter und einem Keratometer gebildet ist. Das Keratometer verfügt über eine Projektionseinrichtung mit einer Placidoscheibe und einem als Kamera ausgebildeten Beobachtungssystem. Das Pachymeter verfügt über eine weitere Projektionseinrichtung, die als eine um eine optische Achse eines Auges rotierende Spaltbeleuchtung ausgebildet ist und die zusammen mit einer Kamera ein nach dem Scheimpflugprinzip arbeitendes, weiteres Beobachtungssystem bildet. Das Pachyeter und das Keratometer sind als zwei separate Apparate ausgebildet, wobei Messdaten beider Geräte in einem externen Computer zur Bestimmung der Dicke der Hornhaut des Auges berechnet werden können.

Ausgehend von diesem Stand der Technik ist es deshalb Aufgabe der vorliegenden Erfindung, ein ophthalmologisches Analysesystem zur Verfügung zu stellen, mit dem die messgenauigkeit und die Messgeschwindigkeit erhöht werden kann.

Diese Aufgabe wird durch ein Analysesystem gemäß der Lehre des Anspruchs 1 gelöst.

Vorteilhafte Ausführungsformen der Erfindung sind Gegenstand der Unteransprüche.

Die Erfindung beruht auf dem Grundgedanken, die bekannten pachymetrischen Analysesysteme in einem Gerät mit einem weiteren Analysesystem zu kombinieren, mit dem die Krümmung des Hornhautgewebes gemessen werden kann. Im Ergebnis gelingt es dadurch während einer einzigen Untersuchung des Patienten an einem Gerät, sowohl die Dicke des Hornhautgewebes als auch die Krümmung des Hornhautgewebes im Messbereich zu ermitteln. Die Krümmung des Hornhautgewebes wird dabei bei der Ableitung des Messwertes für die Dicke des Hornhautgewebes aus den Bildinformationen mitverwendet, so dass Messfehler bei der Ableitung der Dicke des Hornhautgewebes durch Abweichungen der vorausgesetzten Krümmung des Hornhautgewebes ausgeschlossen sind.

Die erste Projektionseinrichtung Untersuchung der Hornhaut ist erfindungsgemäß in der Art einer Spaltbeleuchtung ausgebildet. Die Beleuchtung des Hornhautgewebes mit einem Lichtspalt hat sich Untersuchung der Hornhautdicke bestens bewährt.

Eine besonders hohe Tiefenschärfe der durch das erste Beobachtungssystem aufgenommenen Bilddaten wird erfindungsgemäß dadurch erreicht, dass durch entsprechende Anordnung mit Zwischenwinkeln aus der ersten Projektionseinrichtung und dem ersten Beobachtungssystem ein Scheimpflugsystem gebildet wird.

Weiterhin ist es besonders vorteilhaft, wenn der Strahlengang der Spaltbeleuchtung zumindest einmal an einem Reflektorelement um 90°, insbesondere an zwei Reflektorelementen um jeweils 90°, umgelenkt wird. Auf diese Weise ist es möglich, einen sehr kompakten Geräteaufbau zu realisieren, da der Strahlengang der Spaltbeleuchtung durch entsprechende Umlenkung eingefaltet werden kann.

Eine feststehende Anordnung der Spaltbeleuchtung sorgt für eine hoch genaue Justierung der Spaltblende, so dass Messfehler durch unerwünschte Abweichungen der Spaltblende ausgeschlossen sind.

Erfindungsgemäß bilden die zweite Projektionseinrichtung, das zweite Beobachtungssystem und die Analyseeinrichtung zusammen ein Keratometer.

Zur Messung der Hornhautkrümmung mit dem Keratometer kann eine definierte Messmarke auf die Hornhaut projiziert und die auf der Hornhaut aufgrund der Krümmung auftretende Verzerrung der Messmarke vermessen werden. Besonders geeignet als Messmarke ist dabei die Kombination aus zwei kollimierten Leuchtpunkten und einem im Wesentlichen kreisförmigen, nicht kollimierten Leuchtstreifen.

Zur Erzeugung der kollimierten Leuchtpunkte können am Keratometer zwei beispielsweise zylinderförmige Tuben vorgesehen sein, in deren Innerem als Leuchtmittel eine Leuchtdiode angeordnet ist, wobei der Leuchtdiode zur Erzeugung des kollimierten Lichts zumindest eine Linse vorgeordnet wird.

Zur Erzeugung des kreisförmigen, nicht kollimierten Leuchtstreifens kann ein kreiszylindrisches Lichtleitelement Verwendung finden. Auf der Rückseite des Lichtleitelements, beispielsweise eines Licht leitenden Kunststoffstreifens, wird das Licht an der Stirnseite bzw. am Zylinderumfang durch Beleuchtung mit einem Leuchtmittel eingekoppelt. An der vorderen Stirnseite des Lichtleitelements tritt das Licht dann wieder aus und wird entsprechend der kreiszylindrischen Form des Lichtleitelements als kreisförmiger, nicht kollimierter Leuchtstreifen auf die Hornhaut projiziert. Als Leuchtmittel zur Einkoppelung des Lichtes in das Lichtleitelements können insbesondere Leuchtdioden eingesetzt werden.

Um die durch das erste Beobachtungssystem bzw. zweite Beobachtungssystem beobachteten Bilddaten in einfacher Weise verarbeiten bzw. speichern zu können, ist es besonders vorteilhaft, wenn zur Bildaufnahme entsprechend geeignete Videosensoren eingesetzt werden, die die Bilddaten in Form eines Videosignals an nachgeordnete Funktionseinheiten, beispielsweise ein Bildverarbeitungssystem, weitergeben können.

Um eine digitale Bildverarbeitung zu ermöglichen, sollte das Videosignal vorzugsweise in einem digitalen Datenformat erzeugt oder, soweit die Bilddaten in analoger Form aufgenommen werden, in ein digitales Datenformat umgewandelt werden.

Zur Berechnung der Dicke des Hornhautgewebes bzw. der Krümmung des Hornhautgewebes aus den aufgenommenen Bilddaten sollte vorzugsweise ein digitales Bildverarbeitungssystem eingesetzt werden, das beispielsweise auf einem Standard-PC installiert sein kann.

Als Videosensoren haben sich insbesondere so genannte Chipkameras, beispielsweise CMOS-Chips oder CCD-Chips bewährt.

Zur Messung der Dicke des Hornhautgewebes muss das zu untersuchende Auge möglichst genau relativ zur Beobachtungsoptik ausgerichtet werden. Durch Einsatz des Videosensors des zweiten Beobachtungssystems, das an sich der Messung der Hornhautkrümmung dient, als Einrichtekamera kann eine separate Einrichtekamera eingespart werden.

Die an dem erfindungsgemäßen Analysesystem vorgesehenen Funktionseinheiten machen es weiterhin auch möglich, dieses System zugleich als Pupillometer zur Messung der zeitabhängigen Pupillenreaktion einzusetzen. Abgesehen von einer entsprechend geeigneten Bildverarbeitungssoftware werden zur Durchführung der Pupillometrie keine zusätzlichen optischen Funktionseinheiten benötigt.

Die Pupillenposition könnte insbesondere auch dazu verwendet werden, um die Schnittposition, das heißt die relative Position des zu untersuchenden Auges zum Gerät, zu ermitteln. In dieser Weise kann das Pupillometer also auch als Zentriersystem zur Positionierung des zu untersuchenden Auges eingesetzt werden.

Besonders vorteilhaft ist es, wenn das Analysesystem in einem Gehäuse untergebracht ist, das an einem Geräteträger angekoppelt werden kann. Auf diese Weise kann ein eigenständiger Geräteträger zur Positionierung des Analysesystems vor dem zu untersuchenden Auge entfallen. Statt dessen wird zu diesem Zweck ein Standardgeräteträger, wie er in vielen Augenarztpraxen ohnehin vorhanden ist, verwendet.

Eine Ausführungsform der Erfindung ist in den Zeichnungen schematisch dargestellt und wird nachfolgend beispielhaft erläutert.

Es zeigen:
- **Fig. 1**: ein ophthalmologisches Analysesystem in perspektivischer Ansicht;
- **Fig. 2**: das Analysesystem gemäß **Fig. 1** in seitlicher Ansicht;
- **Fig. 3**: das Analysesystem gemäß **Fig. 1** in Ansicht von oben;
- **Fig. 4**: das Analysesystem gemäß **Fig. 1** in Ansicht von vorn;
- **Fig. 5**: das Analysesystem gemäß **Fig. 1** im Querschnitt entlang der Schnittlinie I-I;

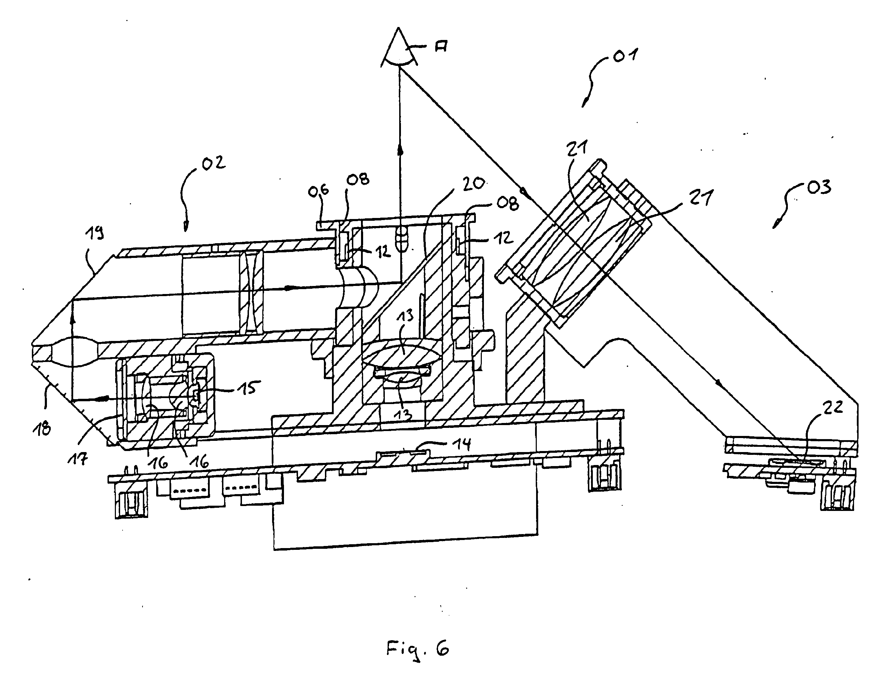

- **Fig. 6**: das Analysesystem gemäß **Fig. 1** im Längsschnitt entlang der Schnittlinie II-II.

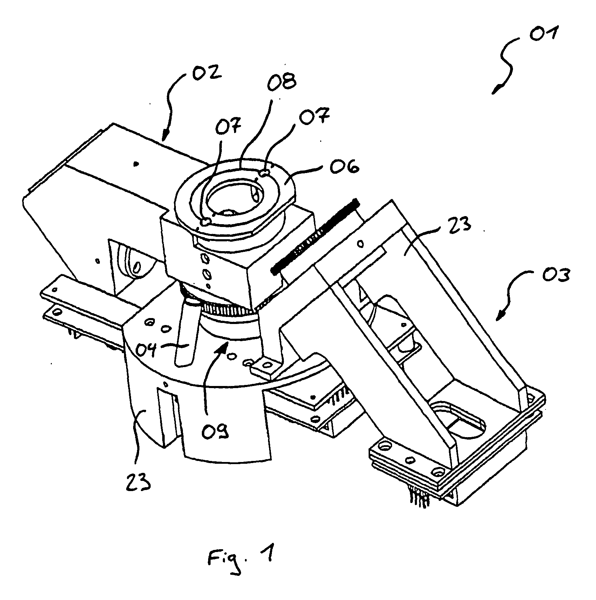

In **Fig. 1** ist ein ophthalmologisches Analysesystem 01 zur kombinierten Untersuchung eines Patientenauges perspektivisch dargestellt. Das Analysesystem 01 ist in ein aus mehreren Teilen zusammengesetztes Gehäuse 23 mit verschiedenen Anbauteilen eingebaut und kann an einem entsprechend geeigneten Geräteträger, der nicht dargestellt ist, angekoppelt werden.

Für die eine Untersuchungsart, bei der die Dicke des Hornhautgewebes des Auges A vermessen wird, ist am Analysesystem 01 eine in der Art einer Spaltbeleuchtung ausgebildete erste Projektionseinrichtung 02 vorgesehen. Die Funktion der ersten Projektionseinrichtung 02 wird nachfolgend noch genauer erläutert. Das Messsystem zur Messung der Hornhautdicke wird durch ein erstes Beobachtungssystem 03, das in der Art eines Scheimpflugkamerasystems ausgebildet ist, vervollständigt.

Das keratometrische Messsystem zur Messung der Krümmung des Hornhautgewebes weist eine Projektionseinrichtung auf, mit der zwei kollimierte Leuchtpunkte und ein im Wesentlichen kreisförmiger, nicht kollimierter Leuchtstreifen auf das Auge A projiziert werden kann. Zur Erzeugung der kollimierten Leuchtpunkte sind zwei auf den Schnittpunkt der verschiedenen Strahlachsen gerichtete Tuben 04 und 05 vorgesehen, in deren Innerem eine Leuchtdiode 10 im Zusammenwirken mit einer Linse 11 einen kollimierten Lichtstrahl erzeugt. Die beiden Lichtstrahlen durchtreten einen Objektivring 06 in zwei dafür vorgesehenen Ausnehmungen 07 und werden auf diese Weise auf das zu untersuchende Auge A proj iziert. Der kreisförmige, nicht kollimierte Leuchtstreifen wird mittels eines kreiszylindrischen Lichtleitelements 08 erzeugt. Das Lichtleitelement 08 wird auf seiner Rückseite mit Leuchtdioden 12 beleuchtet, so dass an der vorderen Stirnseite, dem zu untersuchenden Auge A gegenüberliegend, das in das Lichtleitelement 08 eingekoppelte Licht in der Form eines kreisförmigen Leuchtstreifens austreten kann. Das keratometrische Messsystem wird durch ein zweites Beobachtungssystem 09 vervollständigt.

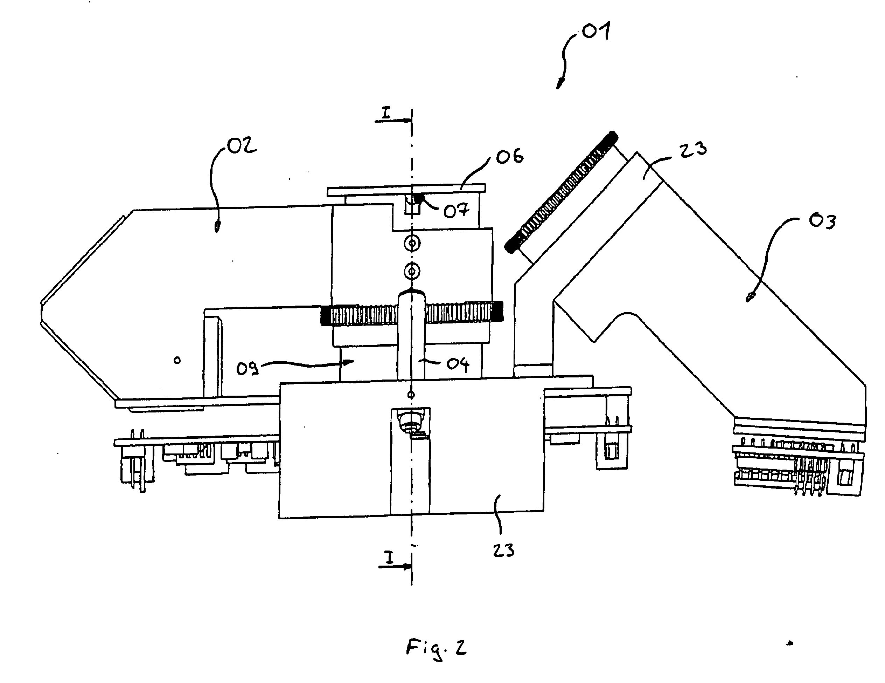

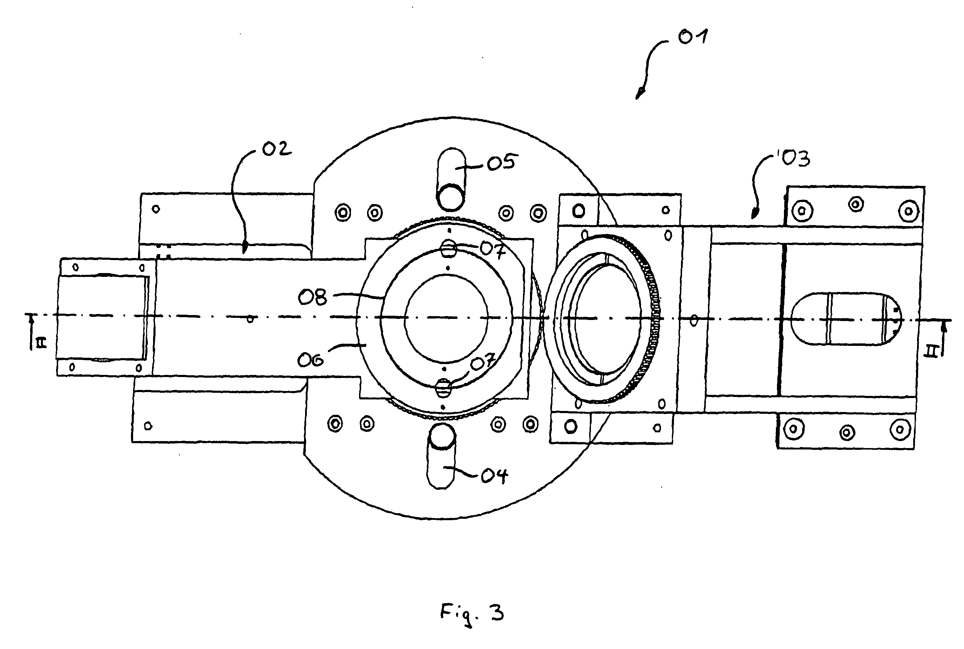

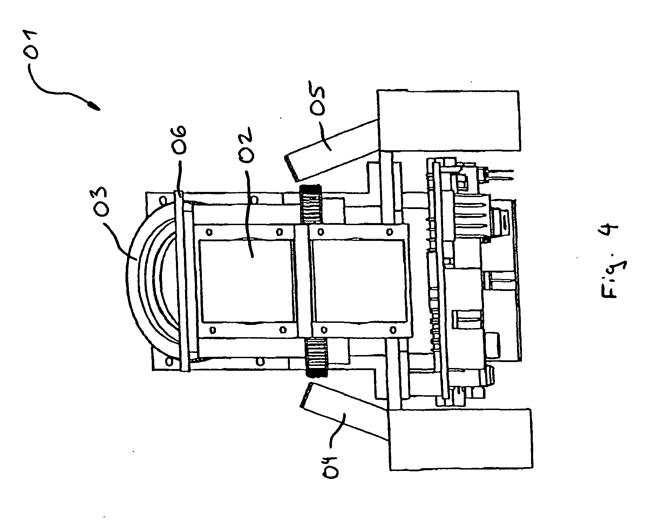

In **Fig. 2****,** **Fig. 3** und **Fig. 4** ist das Analysesystem 01 in verschiedenen Ansichten aus unterschiedlichen Blickwinkeln dargestellt.

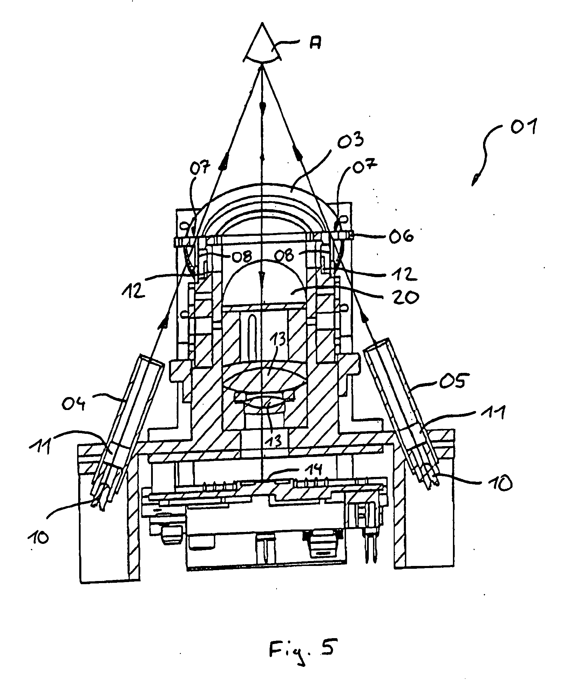

Anhand des Querschnitts in **Fig. 5** soll die Funktionsweise des keratometrischen Messsystems kurz erläutert werden. In den Tuben 04 und 05 ist jeweils eine Leuchtdiode 10 vorgesehen, der jeweils eine Linse 11 vorgeordnet ist. Auf diese Weise werden in den Tuben 04 und 05 kollimierte Lichtstrahlen erzeugt, die durch die Ausnehmungen 07 im Objektivring 06 auf das zu untersuchende Auge A projiziert werden. Zur Erzeugung des kreisringförmigen, nicht kollimierten Leuchtstreifens ist ein kreiszylindrisch geformtes Lichtleitelement 08 in den Objektivring 06 derart eingesetzt, dass die vordere Stirnseite des Lichtleitelements 08 mit der Außenseite des Objektivrings 06 abschließt. Auf der Rückseite des Lichtleitelements 08 ist eine Vielzahl von Leuchtdioden 12 vorgesehen, die gleichmäßig über den Umfang des Lichtleitelements 08 verteilt sind. Das von den Leuchtdioden 12 abgestrahlte Licht wird am Innenumfang in das Lichtleitelement 08 eingekoppelt und tritt an der vorderen Stirnseite des Lichtleitelements 08 als kreisringförmiger, nicht kollimierter Lichtstreifen wieder aus.

Die von den Tuben 04 und 05 und dem Lichtleitelement 08 gemeinsam erzeugte Leuchtmarke wird auf das zu untersuchende Auge A projiziert.

Die Leuchtmarke bildet sich auf der Hornhaut ab und wird durch das Beobachtungssystem 09, das mehreren Linsen 13 und einer Chipkamera 14 aufweist, beobachtet. Die von der Chipkamera 14 aufgenommenen Bilddaten werden digital aufgearbeitet und in ein digitales Bildverarbeitungssystem weitergeleitet. Im Bildverarbeitungssystem werden die Bilddaten in einer Weise ausgewertet, um aus den Bilddaten den Krümmungsradius der Hornhaut zu berechnen.

Im Längsschnitt gemäß **Fig. 7** ist die Funktion des Messsystems zur Messung der Hornhautdicke mit der Projektionseinrichtung 02 und dem Beobachtungssystem 03 schematisch dargestellt. Als Leuchtmittel für die Spaltbeleuchtung in der Projektionseinrichtung 02 dient eine Leuchtdiode 15, der zwei Linsen 16 vorgeordnet sind. Zur Erzeugung des spaltförmigen Lichts ist eine Spaltblende 17 vorgesehen, die einen schmalen Lichtspalt enthält. Anschließend wird der Lichtstrahl an zwei Reflektoren 18 und 19 jeweils um 90° umgelenkt und mittels eines teilverspiegelten Reflektorelements 20 auf das zu untersuchende Auge A projiziert. Der auf das Auge A projizierte Lichtspalt wird durch die Linsen 21 auf eine Chipkamera 22 abgebildet, so dass die dort aufgenommenen digitalen Bilddaten in einem digitalen Bildverarbeitungssystem zur Ableitung der Dicke der Hornhaut ausgewertet werden können.

## Patentansprüche

1. Ophthalmologisches Analysesystem (01) zur Messung der Dicke des Hornhautgewebes an einem zu untersuchenden Auge (A) mit einer ersten Projektionseinrichtung (02), die in der Art einer Spaltbeleuchtung ausgebildet ist, so dass definierte Bereiche des Hornhautgewebes mit einem Lichtspalt beleuchtet werden können, wobei die erste Projektionseinrichtung (02) mit einem ersten Beobachtungssystem (03), durch das die beleuchteten Bereiche des Hornhautgewebes unter einem Winkel relativ zum Strahlengang der ersten Projektionseinrichtung (02) beobachtbar und aufnehmbar sind, derart zusammenwirkt, dass in einer Analyseeinrichtung aus den Bildinformationen des ersten Beobachtungssystems (03) die Dicke des Hornhautgewebes abgeleitet werden kann, wobei der Strahlengang der ersten Projektionseinrichtung (02), ein Strahlengang des ersten Beobachtungssystems (03) und eine Projektionsebene (22) des ersten Bcobachtungssystems (03) derart angeordnet sind, dass die erste Projektionseinrichtung (02) und das erste Beobachtungssystem (03) zusammen ein Scheimpflugsystem bilden, und wobei am Analysesystem (01) eine zweite Projektionseinrichtung (04, 05, 08) vorgesehen ist, mit der definierte Bereiche des Hornhautgewebes beleuchtbar sind, wobei die zweite Projektionseinrichtung (04, 05, 08) mit einem zweiten Beobachtungssystem (09), durch das die beleuchteten Bereiche des Hornhautgewebes beobachtbar und aufnehmbar sind, derart zusammenwirkt, dass in der Analyseeinrichtung aus den Bildinformationen des zweiten Beobachtungssystems (09) die Krümmung des Hornhautgewebes abgeleitet werden kann,
**dadurch gekennzeichnet,**
**dass** die zweite Projektionseinrichtung, das zweite Beobachtungssystem und die Analyseeinrichtung zusammen ein Keratometer bilden, wobei die Analyseeinrichtung eingerichtet ist, die Krümmung des Hornhautgewebes aus den Bildinformationen des zweiten Beobachtungssystems bei der Ableitung der Dicke des Hornhautgewebes aus den Bildinformationen des ersten Beobachtungssystems mit zu verwenden derart, dass Messfehler bei der Ableitung der Dicke des Hornhautgewebes im Messbereich des ersten Beobachtungssystems durch Abweichungen einer vorausgesetzten Krümmung ausgeschlossen sind.

2. Analysesystem nach Anspruch 1,
**dadurch gekennzeichnet,**
**dass** der Strahlengang der Spaltbeleuchtung (02) zumindest einmal an einem Reflektorelement (18, 19) um 90 Grad, insbesondere an zwei Reflektorelementen (18, 19) um jeweils 90 Grad, umgelenkt wird.

3. Analysesystem nach Anspruch 1 oder 2,
**dadurch gekennzeichnet,**
**dass** die Spaltbeleuchtung (02) feststehend angeordnet ist.

4. Analysesystem nach einem der Ansprüche 1 bis 3,
**dadurch gekennzeichnet,**
**dass** als Leuchtmittel der ersten Projektionseinrichtung (02) eine oder mehrere Leuchtdioden (15) dienen.

5. Analysesystem nach Anspruch 1,
**dadurch gekennzeichnet,**
**dass** mit der Projektionseinrichtung (04, 05, 08) des Keratometers eine definierte Messmarke auf die Hornhaut projizierbar ist.

6. Analysesystem nach Anspruch 5,
**dadurch gekennzeichnet,**
**dass** die Messmarke zwei kollimierte Leuchtpunkte und einen im wesentlichen kreisförmigen, nicht kollimierte Leuchtstreifen aufweist.

7. Analysesystem nach Anspruch 6,
**dadurch gekennzeichnet,**
**dass** die kollimierten Leuchtpunkte jeweils von einer in einem Tubus (04, 05) angeordneten Leuchtdiode (10), der zumindest eine Linse (11) vorgeordnet ist, erzeugt werden.

8. Analysesystem nach Anspruch 6 oder 7,
**dadurch gekennzeichnet,**
**dass** der kreisförmige, nicht kollimierte Leuchtstreifen von einem kreiszylindrischen Lichtleiterelement (08) erzeugt wird, wobei das Licht von zumindest einem Leuchtmittel (15) an der hinteren Stirnseite und/oder am Zylinderumfang ins Lichtleiterelement eingekoppelt wird und an der vorderen Stirnseite aus dem Lichtleiterelement (08) austritt.

9. Analysesystem nach Anspruch 8,
**dadurch gekennzeichnet,**
**dass** als Leuchtmittel mehrere über den Umfang des kreiszylindrischen Lichtleiterelements (08) verteilte Leuchtdioden (12) dienen.

10. Analysesystem nach einem der Ansprüche 1 bis 9,
**dadurch gekennzeichnet,**
**dass** im ersten Beobachtungssystem (03) und/oder im zweiten Beobachtungssystem (09) jeweils zumindest ein Videosensor (14, 22) vorgesehen ist, mit dem die Hornhaut beobachtet und aufgenommen werden kann, wobei der Videosensor (14, 22) die Bilddaten in Form eines Videosignal weitergibt.

11. Analysesystem nach Anspruch 10,
**dadurch gekennzeichnet,**
**dass** das Videosignal in einem digitalem Datenformat erzeugt oder in ein digitales Datenformat umgewandelt wird.

12. Analysesystem nach Anspruch 11,
**dadurch gekennzeichnet,**
**dass** als Analyseeinrichtung ein digitales Bildverarbeitungssystem vorgesehen ist, mit dem aus den digitalen Bilddaten die Dicke des Hornhautgewebes und/oder die Krümmung des Hornhautgewebes abgeleitet werden kann.

13. Analysesystem nach einem der Ansprüche 10 bis 12,
**dadurch gekennzeichnet,**
**dass** der Videosensor in der Art einer Chipkamera (14, 22) ausgebildet ist.

14. Analysesystem nach einem der Ansprüche 10 bis 13,
**dadurch gekennzeichnet,**
**dass** der Videosensor (14) des zweiten Beobachtungssystems (09) als Einrichtkamera zur lagerichtigen Ausrichtung des zu untersuchenden Auges (A) einsetzbar ist.

15. Analysesystem nach einem der Ansprüche 1 bis 14,
**dadurch gekennzeichnet,**
**dass** das Analysesystem (01) als Pupillometer einsetzbar ist.

16. Analysesystem nach Anspruch 15,
**dadurch gekennzeichnet,**
**dass** das Pupillometer als Zentriersystem zur Positionierung des zu untersuchenden Auges (A) einsetzbar ist.

17. Analysesystem nach einem der Ansprüche 1 bis 16,
**dadurch gekennzeichnet,**
**dass** das Analysesystem (01) in einem Gehäuse (23) untergebracht ist, das an einem Geräteträger ankoppelbar ist.

## Claims

1. An ophthalmological analysis system (01) for measuring the thickness of the corneal tissue on an eye (A) to be examined, said system comprising a first projection device (02), which is designed in the manner of a slit illumination device so that defined areas of the corneal tissue can be illuminated by a light slit, the first projection device (02) cooperating with a first monitoring system (03), by which the illuminated areas of the corneal tissue can be monitored and recorded at an angle to the beam path of the first projection device (02), in such a way that the thickness of the corneal tissue can be derived in an analysis device from the image information of the first monitoring system (03), the beam path of the first projection device (02), a beam path of the first monitoring system (03) and a projection plane (22) of the first monitoring system (03) being arranged in such a way that the first projection device (02) and the first monitoring system (03) together form a Scheimpflug system, and a second projection device (04, 05, 08) being provided on the analysis system (01), said second projection device (04, 05, 08) being able to illuminate defined areas of the corneal tissue and cooperating with a second monitoring system (09), with which the illuminated areas of the corneal tissue can be monitored and recorded, in such a way that the curvature of the corneal tissue can be derived in the analysis device from the image information of the second monitoring system (09),
**characterised in that**
the second projection device, the second monitoring system and the analysis device together form a keratometer, the analysis device being designed to use the curvature of the corneal tissue from the image information of the second monitoring system when deriving the thickness of the corneal tissue from the image information of the first monitoring system in such a way that measurement errors occurring when deriving the thickness of the corneal tissue in the measurement field of the first monitoring system and caused by deviations of an expected curvature are eliminated.

2. The analysis system according to claim 1,
**characterised in that**
the beam path of the gap illumination device (02) is deflected at least once at a reflector element (18, 19) by 90 degrees, in particular at two reflector elements (18, 19) by 90 degrees in each case.

3. The analysis system according to either claim 1 or claim 2,
**characterised in that**
the gap illumination device (02) is arranged in a fixed manner.

4. The analysis system according to any one of claims 1 to 3,
**characterised in that**
one or more LEDs (15) are used as lamps of the first projection device (02).

5. The analysis system according to claim 1,
**characterised in that**
a defined reference mark can be projected onto the cornea by means of the projection device (04, 05, 08) of the keratometer.

6. The analysis system according to claim 5,
**characterised in that**
the reference mark comprises two collimated light dots and a substantially circular, uncollimated light strip.

7. The analysis system according to claim 6,
**characterised in that**
the collimated light dots are each produced by an LED (10) which is arranged in a lens tube (04, 05) at least one lens (11) being arranged in front of said LED.

8. The analysis system according to either claim 6 or claim 7,
**characterised in that**
the circular, uncollimated light strip is produced by a circular cylindrical optical waveguide element (08), the light from at least one lamp (15) being coupled into the optical waveguide element at the rear end face and/or at the circumference of the cylinder and exiting from the optical waveguide element (08) at the front end face.

9. The analysis system according to claim 8,
**characterised in that**
a plurality of LEDs (12) distributed over the circumference of the circular cylindrical optical waveguide element (08) are used as lamps.

10. The analysis system according to any one of claims 1 to 9,
**characterised in that**
at least one video sensor (14, 22) is provided in the first monitoring system (03) and/or in the second monitoring system (09), it being possible to monitor and record the cornea by means of said video sensor, the video sensor (14, 22) relaying the image data in the form of a video signal.

11. The analysis system according to claim 10,
**characterised in that**
the video signal is produced in a digital data format or is converted into a digital data format.

12. The analysis system according to claim 11,
**characterised in that**
a digital image processing system is provided as the analysis device, it being possible to device the thickness of the corneal tissue and/or the curvature of the corneal tissue from the digital image data by means of said image processing system.

13. The analysis system according to any one of claims 10 to 12,
**characterised in that**
the video sensor is designed in the manner of a chip camera (14, 22).

14. The analysis system according to any one of claims 10 to 13,
**characterised in that**
the video sensor (14) of the second monitoring system (09) can be used as an adjusting camera to orientate, in a positionally correct manner, the eye (A) which is to be examined.

15. The analysis system according to any one of claims 1 to 14,
**characterised in that**
the analysis system (01) can be used as a pupillometer.

16. The analysis system according to claim 15,
**characterised in that**
the pupillometer can be used as a centring system to position the eye (A) which is to be examined.

17. The analysis system according to any one of claims 1 to 16,
**characterised in that**
the analysis system (01) is accommodated in a housing (23), which can be coupled to an apparatus support.

## Revendications

1. Système d'analyse ophtalmologique (01) pour mesurer l'épaisseur du tissu cornéen sur un oeil (A) à examiner, comprenant un premier équipement de projection (02) réalisé à la manière d'un dispositif d'éclairage à fente, de sorte que des zones définies du tissu cornéen puissent être éclairées par une fente lumineuse, dans lequel le premier équipement de projection (02) coopère avec un premier système d'observation (03), qui permet d'observer et d'enregistrer les zones éclairées du tissu cornéen sous un angle relatif à la trajectoire du faisceau du premier équipement de projection (02), de telle sorte que dans un équipement d'analyse, l'épaisseur du tissu cornéen peut être déduite des informations d'image du premier système d'observation (03), dans lequel la trajectoire du faisceau du premier équipement de projection (02), une trajectoire du faisceau du premier système d'observation (03) et un plan de projection (22) du premier système d'observation (03) sont disposés de telle sorte que le premier équipement de projection (02) et le premier système d'observation (03) constituent ensemble un système de Scheimpflug, et dans lequel, sur le système d'analyse (01), un deuxième équipement de projection (04, 05, 08) est prévu qui permet d'éclairer des zones définies du tissu cornéen, dans lequel le deuxième équipement de projection (04, 05, 08) coopère avec un deuxième système d'observation (09), qui permet d'observer et d'enregistrer les zones éclairées du tissu cornéen, de telle sorte que dans l'équipement d'analyse, la courbure du tissu cornéen peut être déduite des informations d'image du deuxième système d'observation (09),
**caractérisé en ce que**
le deuxième équipement de projection, le deuxième système d'observation et l'équipement d'analyse constituent ensemble un kératomètre, dans lequel l'équipement d'analyse est agencé pour utiliser également la courbure du tissu cornéen issue des informations d'image du deuxième système d'observation lors de la dérivation de l'épaisseur du tissu cornéen des informations d'image du premier système d'observation, de façon à exclure lors de la dérivation de l'épaisseur du tissu cornéen dans la plage de mesure du premier système d'observation des erreurs de mesure dues à des écarts d'une courbure prédéfinie.

2. Système d'analyse selon la revendication 1,
**caractérisé en ce que**
la trajectoire du faisceau de l'éclairage à fente (02) est déviée de 90 degrés, au moins une fois au niveau d'un élément réfléchissant (18, 19), en particulier de 90 degrés respectivement au niveau de deux éléments réfléchissants (18, 19).

3. Système d'analyse selon la revendication 1 ou 2,
**caractérisé en ce que**
l'éclairage à fente (02) est disposé de façon fixe.

4. Système d'analyse selon l'une quelconque des revendications 1 à 3,
**caractérisé en ce qu'**
une ou plusieurs diodes électroluminescentes (15) servent de moyens lumineux du premier équipement de projection (02).

5. Système d'analyse selon la revendication 1,
**caractérisé en ce que**
l'équipement de projection (04, 05, 08) du kératomètre permet de projeter sur la cornée un repère de mesure défini.

6. Système d'analyse selon la revendication 5,
**caractérisé en ce que**
le repère de mesure présente deux points lumineux collimatés et une bande lumineuse non collimatée, substantiellement circulaire.

7. Système d'analyse selon la revendication 6,
**caractérisé en ce que**
les points lumineux collimatés sont générés par une diode électroluminescente (10) disposée dans un tube (04, 05) et en amont de laquelle se trouve une lentille (11).

8. Système d'analyse selon la revendication 6 ou 7,
**caractérisé en ce que**
la bande lumineuse non collimatée circulaire est générée par un élément conducteur optique cylindrique circulaire (08), dans lequel la lumière est injectée dans l'élément conducteur optique par au moins un moyen lumineux (15) au niveau de la face arrière et/ou de la circonférence cylindrique et sort de l'élément conducteur optique (08) au niveau de la face avant.

9. Système d'analyse selon la revendication 8,
**caractérisé en ce que**
plusieurs diodes électroluminescentes (12) réparties sur la circonférence de l'élément conducteur optique (08) cylindrique circulaire servent de moyens lumineux.

10. Système d'analyse selon l'une quelconque des revendications 1 à 9,
**caractérisé en ce que**
dans le premier système d'observation (03) et/ou dans le deuxième système d'observation (09), respectivement au moins un capteur vidéo (14, 22) est prévu qui permet d'observer et d'enregistrer la cornée, le capteur vidéo (14, 22) reproduisant les données d'image sous la forme d'un signal vidéo.

11. Système d'analyse selon la revendication 10,
**caractérisé en ce que**
le signal vidéo est généré dans un format de données numérique ou converti en un format de données numérique.

12. Système d'analyse selon la revendication 11,
**caractérisé en ce qu'**
un système de traitement d'image numérique est prévu comme équipement d'analyse permettant de déduire l'épaisseur du tissu cornéen et/ou la courbure du tissu cornéen des données d'image numériques.

13. Système d'analyse selon l'une quelconque des revendications 10 à 12,
**caractérisé en ce que**
le capteur vidéo est réalisé à la manière d'une caméra sur puce (14, 22).

14. Système d'analyse selon l'une quelconque des revendications 10 à 13,
**caractérisé en ce que**
le capteur vidéo (14) du deuxième système d'observation (09) peut être mis en oeuvre comme une caméra de mise au point pour aligner l'oeil (A) à examiner dans la bonne position.

15. Système d'analyse selon l'une quelconque des revendications 1 à 14,
**caractérisé en ce que**
le système d'analyse (01) est utilisable comme pupillomètre.

16. Système d'analyse selon la revendication 15,
**caractérisé en ce que**
le pupillomètre est utilisable comme système de centrage pour positionner l'oeil (A) à examiner.

17. Système d'analyse selon l'une quelconque des revendications 1 à 16,
**caractérisé en ce que**
le système d'analyse (01) est logé dans un boîtier (23) qui peut être couplé avec un support d'appareil.
